(19) Europäisches Patentamt
European Patent Office
Office européen des brevets

(11) Numéro de publication : **0 128 059 B1**

(12) # FASCICULE DE BREVET EUROPÉEN

(45) Date de publication du fascicule du brevet : **28.05.86**

(51) Int. Cl.[4] : **C 07 C 31/38**, C 07 C 29/136

(21) Numéro de dépôt : **84400856.5**

(22) Date de dépôt : **27.04.84**

(54) **Procédé de préparation de 1,1-dihydro-perfluoroalcanols.**

(30) Priorité : **06.05.83 FR 8307569**

(43) Date de publication de la demande : **12.12.84 Bulletin 84/50**

(45) Mention de la délivrance du brevet : **28.05.86 Bulletin 86/22**

(84) Etats contractants désignés : **AT BE CH DE FR GB IT LI LU NL SE**

(56) Documents cités :
**FR-A- 2 060 357**
**FR-A- 2 183 981**
**US-A- 4 273 947**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Cordier, Georges**
**50, Chemin des Hermières**
**F-69340 Francheville (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

**EP 0 128 059 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet un procédé de préparation de 1,1-dihydro perfluoroalcanols ; elle concerne plus particulièrement un procédé de préparation de 1,1-dihydro-perfluoroalcanols par hydrogénation des acides aliphatiques perfluorés correspondants.

On connaît dans l'art antérieur la demande de brevet français 70.31913 publiée sous le n° 2.060.357 qui décrit la préparation de dihydro-1,1 perfluoroalcanols répondant à la formule générale $C_nF_{2n+1}$. $CH_2OH$ dans laquelle $n \geq 3$ par hydrogénation d'acides ou d'esters de formule $C_nF_{2n+1}COOR$ où R représente un atome d'hydrogène ou un radical alkyle et où $n \geq 3$ en présence d'un catalyseur au ruthénium.

Selon ce procédé on effectue l'hydrogénation en présence de 4 à 1 000 % en poids d'eau par rapport à l'acide ou à l'ester et sous température et pression d'hydrogène élevées. La température est comprise entre 80 et 240 °C et la pression d'hydrogène initiale varie entre 5 et 700 kgp/cm$^2$.

Ce procédé ne prévoit pas la possibilité de mettre en œuvre l'acide trifluoroacétique (n = 1) pour obtenir le trifluoroéthanol.

La préparation de ce même trifluoroéthanol n'est pas non plus envisagée dans le brevet des Etats-Unis d'Amérique 2.862.977 qui décrit la préparation de dihydro-1,1 perfluoroalkylalcools de formule $C_nF_{2n+1}CH_2OH$ ($n > 1$) par hygrogénation de l'acide correspondant sous une pression d'au moins 1 000 p.s.i. (70 bars) et à une température supérieure à 150 °C en présence d'un catalyseur au ruthénium.

On connaît encore le brevet des Etats-Unis d'Amérique 4.273.947 qui décrit un procédé pour hydrogéner l'acide trifluoroacétique ($CF_3COOH$) selon lequel on met en contact cet acide en phase liquide avec de l'hydrogène en présence d'un catalyseur supporté ou non supporté à base de rhodium ou d'irridium sous une pression de 5 à 15 atmosphères et à une température de 50 à 150 °C.

En étudiant les procédés de l'art antérieur, ci-dessus analysés, la demanderesse a constaté que dans le cas de l'hydrogénation de l'acide trifluoroacétique il se développe des réactions secondaires d'hydrogénolyse qui donnent naissance à des produits gazeux comme HF, $CF_3—CH_3$, $CH_3—CH_3$ et $CH_4$ qui, s'accumulant dans le réacteur, bloquent la réaction d'hydrogénation. Ces réactions secondaires existent aussi dans le cas des acides ayant un nombre d'atomes de carbone plus élevé mais, dans ce cas, les produits secondaires ont une tension de vapeur beaucoup plus faible et n'affectent pas aussi négativement la réaction d'hydrogénation. La demanderesse a de plus constaté que ces réactions secondaires étaient plus importantes dans le cas d'un catalyseur au ruthénium que dans le cas d'un catalyseur au rhodium.

La Demanderesse a maintenant découvert un procédé permettant de minimiser et même d'annuler ces réactions secondaires qui sont également à l'origine d'une perte de rendement non négligeable.

L'invention a donc pour objet un procédé de préparation de 1,1-dihydro-perfluoroalcanols par hydrogénation des perfluoroacides aliphatiques correspondants, caractérisé en ce que l'on fait réagir un acide de formule :

$$CnF_{2n+1}COOH$$

dans laquelle n est supérieur ou égal à 1, avec de l'hydrogène, en présence d'un catalyseur à base de ruthénium, rhodium, irridium ou platine sous une pression totale comprise entre 1 et 50 bars et en présence d'au moins un additif choisi parmi le groupe comprenant les anions halogénures et les cations métalliques des colonnes IB, IIB, IVA, VA et VB de la classification périodique des éléments.

On met en œuvre, de préférence, une quantité d'additif comprise entre 0,001 et 10 atomes-gramme par atome-gramme de métal catalyseur c'est-à-dire par atome-gramme de ruthénium, rhodium, iridium ou platine. Encore plus préférentiellement cette quantité est comprise entre 0,01 et 1 atome-gamme.

Le cation métallique est choisi de préférence parmi les cations des métaux des colonnes IB, IIB, IVA, VA et VB de la classification périodique des éléments. Encore plus préférentiellement il dérive des métaux suivants : Cu, Ag, Cd, Hg, Sn, Pb, Sb, Bi et Ta. On préfère tout particulièrement mettre en œuvre les cations dérivés du Pb et du Ta.

Le cation métallique est mis en œuvre de préférence sous forme d'un sel ou d'un oxyde. On peut citer comme exemples : le pentoxyde de Tantale $Ta_205$, l'oxyde de bismuth $Bi_203$, d'étain $SnO_2$, les acétates et trifluoracétates de $Pb^{2+}$, $Cu^{2+}$, $Hg^+$, $Cd^{2+}$ ou encore les carbonates ($Ag_2CO_3$) et les sulfates de $Cu^{2+}$, $Cd^{2+}$.

L'anion halogénure est mis en œuvre de préférence sous forme de l'acide hydrogéné correspondant (HCl, HF, HBr et HI) ou de l'halogénure des métaux des colonnes IB, IIB, IVA, VA et VB précités ou encore de l'halogénure des métaux alcalins et alcalinoterreux des colonnes IA ou IIA de la classification périodique. On peut citer comme exemples : les fluorures, chlorures, bromures, iodures de lithium, sodium, potassium, calcium, magnésium.

On préfère tout particulièrement utiliser les acides hydrogénés.

L'invention est particulièrement bien adaptée à la réalisation de la réaction d'hydrogénation sous une pression comprise entre 5 et 30 bars.

Les catalyseurs mis en œuvre dans le cadre du procédé selon l'invention peuvent être sous forme

métallique proprement dite, sous forme d'un oxyde ou d'un sel ou un mélange d'entre eux. Ils peuvent être, de plus, déposés sur support ou pas.

On peut utiliser tout support inerte dans les conditions de la réaction comme le charbon, la silice ou l'alumine. On préfèrera tout particulièrement utiliser le charbon.

La quantité de métal déposée sur le support est de préférence comprise entre 0,1 et 20 % en poids, plus préférentiellement entre 1 et 10 %.

On peut citer comme exemples de catalyseurs : les ruthénium, rhodium, irridium ou platine sous forme métallique, leurs oxydes, hydroxydes et sels d'acides (nitrates, sulfates et acétates) et des mélanges d'entre eux éventuellement déposés sur charbon ou silice.

Selon un mode tout particulièrement préféré de réalisation on utilise du rhodium métallique déposé à 5 % sur charbon.

On opère de préférence à une température comprise entre 20 et 200°. Encore plus préférentiellement la température est comprise entre 70 et 150 °C.

La quantité de catalyseur (exprimée en % du métal) mise en œuvre est de préférence inférieure à 10 % en poids par rapport au perfluoroacide aliphatique. Encore plus préférentiellement on utilise moins de 2 % de catalyseur.

La réaction a lieu, de préférence, en présence d'un solvant. Toutefois, il n'est pas exclu d'opérer sans solvant. On peut utiliser de l'eau ou un composé organique inerte (alcane par exemple). L'eau facilite la mise en œuvre de catalyseurs qui généralement sont vendus humides.

L'invention est particulièrement intéressante pour l'obtention du trifluoroéthanol par hydrogénation de l'acide trifluoracétique.

D'autres avantages et caractéristiques de l'invention apparaîtront à la lecture des exemples qui suivent :

Exemple 1

Dans un autoclave en Hastelloy $B_2$ résistant à la corrosion, de 125 ml de volume utile, on charge :
0,3 mole d'acide trifluoroacétique
0,3 g de catalyseur à 5 % de Rh déposé sur un charbon de grande surface spécifique $\geqslant 900\ m^2g^{-1}$
0,018 g de pentoxyde de Tantale soit un rapport Ta/Rh = 0,6

Après élimination de l'air par un gaz inerte tel que l'azote puis remplacement de l'azote par de l'hydrogène on porte la pression du réacteur à 45 bars et on chauffe à 120 °C. La réaction s'ammorce pendant la montée en température et de ce fait la consommation d'hydrogène compense l'augmentation de pression liée à la dilatation des gaz. On alimente alors en continu l'hydrogène nécessaire à la réaction en maintenant la pression constante à 45 bars. La réaction stoppe après 2 heures 10 minutes.

Après refroidissement et dégazage de l'enceinte l'analyse de la masse réactionnelle par CPV montre que le taux de transformation de l'acide trifluoroacétique est de 100 % et le rendement en trifluoroéthanol est > 99,5 %. L'analyse des gaz contenus dans le réacteur permet de calculer un rendement de 0,3 % en méthane.

Le rendement en $CF_3CH_3$ et en $C_2H_6$ est nul.

Si on n'ajoute pas de pentoxyde de Tantale, le rendement en trifluoroéthanol n'est plus que de 94 % tandis que le rendement en $CF_3CH_3$ est de 5 % et celui de $CH_4$ de 0,8 %.

Exemple 2

On répète l'exemple 1 en ajoutant non plus du pentoxyde de Tantale, mais 0,27 mg d'acétate de plomb $Pb(CH_3COO)_2$, $3H_2O$ soit un rapport Pb/Rh = 0,005. La réaction est stoppée après 200 minutes. Le taux de transformation de l'acide trifluoroacétique est de 97,5 %, le rendement de l'acide trifluoroacétique est de 97,5 %, le rendement en trifluoroéthanol est de 98 %. Le rendement en $CF_3CH_3$ est de 1,2 % et celui du méthane de 0,06 %.

Exemple 3

On répète l'exemple 2 en chargeant 10 fois plus de $Pb(CH_3COO)_2$, $3H_2O$.

La réaction stoppe après 500 minutes. Le taux de transformation de l'acide trifluoroacétique est de 96,5 % et le rendement en trifluoroéthanol est quantitatif. Il n'y a plus de gaz fluorés lourds et on ne trouve que des traces de méthane.

Exemple 4

On opère dans le même réacteur que dans l'exemple 1, mais on charge :
0,2 mole d'acide trifluoroacétique
0,4 g du catalyseur Rh à 5 % (charbon employé à l'exemple 1)
2,5 ml d'eau et pas d'additif.

La réaction est conduite comme il est dit à l'exemple 1 mais à 150 °C/20 bars de pression totale. Elle

stoppe après 85 minutes. Le taux de transformation de l'acide trifluoroacétique est de 99,3 %. Le rendement en trifluoroéthanol est de 93,5 %.

L'analyse des gaz de l'enceinte permet de calculer un rendement de 5 % pour $CF_3CH_3$ ; 0,4 % pour $CF_2HCH_3$, 0,4 % pour $C_2H_6$ et 0,4 % pour $CH_4$ soit un rendement total en sous-produit de 6,2 %.

Exemple 5

On répète l'exemple 4 en ajoutant au milieu réactionnel 20 mg d'acétate de cuivre $Cu(CH_3COO)_2$, $H_2O$ soit un rapport $Cu^{2+}/Rh$ 0,5. La réaction stoppe après 2 heures à 150 °C sous 20 bars de pression totale.

Le taux de transformation de l'acide trifluoroacétique est de 48,6 % et le rendement en trifluoroéthanol est de 97,5 %.

L'analyse des gaz permet de calculer un rendement en $CF_3CH_3$ de 2,1 %, tandis que ceux de $CF_2HCH_3$ ; $C_2H_6$ et $CH_4$ sont chacun égaux à 0,2 %.

Exemple 6

On répète l'exemple 5 en remplaçant l'acétate cuivrique par 0,003 g de sulfate stanneux $Sn(SO_4)$ soit $Sn^{2+}/Rh$ : 0,07. Après 6 heures 50 minutes de réaction à 150 °C sous 20 bars de pression, la réaction stoppe. Le taux de transformation de l'acide trifluoroacétique est de 94 %, tandis que le rendement en trifluoroéthanol est de 97,2 %.

L'analyse des gaz montre un rendement en $CF_3CH_3$ de 2,1 % et des traces de $CF_2HCH_3$ (0,05 %) et d'éthane 0,15 %.

Exemple 7

On répète l'exemple 5 en ajoutant au milieu réactionnel 0,016 ml d'une solution aqueuse d'HI à 1 mole/l.

La réaction stoppe après 355 minutes. Le taux de transformation de l'acide trifluoroacétique est de 96,3 %. Le rendement en trifluoroéthanol est de 96 %. L'analyse des gaz permet de calculer

| | |
|---|---|
| — rendement en $CF_3CH_3$ | 3 % |
| — rendement en $CF_2HCH_3$ | 0,2 % |
| — rendement en $C_2H_6$ | 0,3 % |
| — rendement en $CH_4$ | 0,3 % |

Exemple 8

On répète l'exemple 5 en ajoutant au milieu 0,16 ml d'une solution aqueuse d'HCl à 1 mole/l.

Après 70 minutes de réaction l'analyse du milieu réactionnel indique que le taux de transformation de l'acide trifluoroacétique est de 40 % et le rendement en trifluoroéthanol de 98,5 %.

L'analyse des gaz permet de calculer :

| | |
|---|---|
| — rendement en $CF_3CH_3$ | 1 % |
| — rendement en $CF_2HCH_3$ | 0,3% |
| — rendement en $C_2H_6$ | 0 % |
| — rendement en $CH_4$ | 0,2 % |

Exemple 9

On opère comme il est dit dans l'exemple 1 mais le catalyseur est maintenant du ruthénium déposé à 5 % sur un charbon de grande surface spécifique (⩾ 900 $m^2/g$) et les charges mises en œuvre sont

| | |
|---|---|
| — acide trifluoroacétique | 0,2 mole |
| — catalyseur Ru à 5 %/C | 2,5 g |
| — $H_2O$ | 7,5 g |

La réaction est conduite à 150 °C sous 50 bars de pression totale. Elle stoppe après 30 minutes.

L'analyse de la masse réactionnelle indique que la transformation de l'acide trifluoroacétique est totale. Le rendement en trifluoroéthanol est de 90,5 %. L'analyse de la phase gazeuse indique les rendements suivants :

| | |
|---|---|
| — $CF_3CH_3$ | 6,7 % |
| — $CHF_2CH_3$ | 0 % |
| — $C_2H_6$ | 1,9 % |
| — $CH_4$ | 0,9 % |

Exemple 10

On répète l'exemple 9 avec 2 fois moins du même catalyseur en ajoutant au milieu 0,481 g de sulfate de Cadmium ($3CdSO_4$, $8H_2O$) soit un rapport Cd/Ru 4.

La réaction stoppe après 275 minutes. Le taux de transformation de l'acide trifluoroacétique est de 94,5 % tandis que le rendement en trifluoroéthanol est de 95,3 %. L'analyse de la phase gazeuse permet de calculer les rendements suivants :

| | |
|---|---|
| — $CF_3CH_3$ | 2,6 % |
| — $CF_2HCH_3$ | 0,8 % |
| — $C_2H_6$ | 0,8 % |
| — $CH_4$ | 0,3 % |

Exemple 11

On répète l'exemple 10 en ajoutant au milieu à la place du sulfate de cadmium 0,195 g de sulfate d'argent $Ag_2SO_4$, soit un rapport Ag/Ru = 2.

La réaction stoppe après 90 minutes. Le taux de transformation de l'acide trifluoroacétique est de 97 % et le rendement en trifluoroéthanol est de 93,5 %.

L'analyse de la phase gazeuse permet de calculer les rendements suivants :

| | |
|---|---|
| — $CF_3CH_3$ | 4,2 % |
| — $CF_2HCH_3$ | 0,65 % |
| — $C_2H_6$ | 1,0 % |
| — $CH_4$ | 0,3 % |

Exemple 12

On répète l'exemple 10 en remplaçant le sulfate de Cadmium par du trioxyde d'antimoine $Sb_2O_3$ (0,091 g) soit un rapport Sb/Ru = 1.

La réaction stoppe après 255 minutes. Le taux de transformation de l'acide trifluoroacétique est de 52,5 % et le rendement en trifluoroéthanol est de 96,3 %.

L'analyse de la phase gazeuse permet de calculer les rendements suivants :

| | |
|---|---|
| — $CF_3CH_3$ | 2,45 % |
| — $CF_2HCH_3$ | 0,3 % |
| — $C_2H_6$ | 0,3 % |
| — $CH_4$ | 0,3 % |

Exemple 13

On répète l'exemple 10 en remplaçant le sulfate de cadmium par 0,155 g de sulfate mercureux soit un rapport Hg/Ru = 1.

La réaction stoppe après 150 minutes.

Le taux de transformation de l'acide trifluoroacétique est de 52,5 % et le rendement en trifluoroéthanol est de 96,5 %.

Dans le même temps les rendements calculés sont pour les sous-produits gazeux :

| | |
|---|---|
| — $CF_3CH_3$ | 2,25 % |
| — $CF_2HCH_3$ | 0 % |
| — $C_2H_6$ | 0,85 % |
| — $CH_4$ | 0,35 % |

Exemple 14

On répète l'exemple 10 en remplaçant le sulfate de cadmium par 47 mg d'acétate de plomb soit Pb/Ru = 0,2.

La réaction stoppe après 450 minutes.

Le taux de transformation de l'acide trifluoroacétique est de 97,9 %. Le rendement en trifluoroéthanol est de 95,8 % alors que les rendements en sous-produits gazeux sont :

| | |
|---|---|
| — $CF_3CH_3$ | 2,6 % |
| — $C_2H_6$ | 1,3 % |
| — $CH_4$ | 0,3 % |

Exemple 15

On répète l'exemple 10 en remplaçant le sulfate de Cadmium par 250 mg d'acétate cuivrique hydraté $Cu(CH_3COO)_2$, $H_2O$ soit un rapport $Cu^{2+}/Ru$ = 2.

La réaction stoppe après 815 minutes.

Le taux de transformation de l'acide trifluoroacétique est de 98,3 % tandis que le rendement en trifluoroéthanol est de 97,1 %.

Les rendements en sous-produits gazeux sont :

| | |
|---|---|
| — $CF_3CH_3$ | 2,0 % |
| — $C_2H_6$ | 0,55 % |
| — $CH_4$ | 0,3 % |

Exemple 16

On répète l'exemple 10 en remplaçant le sulfate de cadmium par 0,5 ml d'une solution aqueuse d'HCl à 0,1 mole/l et on conduit la réaction à 140 °C sous une pression de 37 bars.

Après 215 minutes la réaction stoppe.

Le taux de transformation de l'acide trifluoroacétique est de 98,4 %. Le rendement en trifluoroéthanol est de 94,5 %.

Les rendements en sous-produits gazeux sont :

| | |
|---|---|
| — $CF_3CH_3$ | 3,9 % |
| — $C_2H_6$ | 1,9 % |
| — $CH_4$ | 0,35 % |

**Revendications**

1. Procédé de préparation de 1,1-dihydro-perfluoroalcanols par hydrogénation des perfluoroacides aliphatiques correspondants, caractérisé en ce que l'on fait réagir un acide de formule :

$$CnF_{2n+1}COOH$$

dans laquelle n est supérieur ou égal à 1, avec de l'hydrogène, en présence d'un catalyseur à base de ruthénium, rhodium, irridium ou platine sous une pression totale comprise entre 1 et 50 bars, à une température comprise entre 20° et 200 °C, et en présence d'au moins un additif choisi parmi le groupe comprenant les anions halogénure et les cations métalliques des colonnes IB, IIB, IVA, VA et VB de la classification périodique des éléments.

2. Procédé selon la revendication 1, caractérisé en ce que l'on utilise une quantité d'additif comprise entre 0,001 et 10 atomes-gramme par atome-gramme de métal catalyseur.

3. Procédé selon la revendication 2, caractérisé en ce que la quantité d'additif est comprise entre 0,01 et 1 atome-gramme.

4. Procédé selon l'une quelconque des revendications précédentes, caractérisé en ce que le cation métallique est choisi parmi les cations des métaux des colonnes IB, IIB, IVA, VA et VB de la classification périodique.

5. Procédé selon la revendication 4, caractérisé en ce que le cation métallique est choisi parmi Cu, Ag, Cd, Hg, Sn, Pb, Sb, Bi et Ta.

6. Procédé selon la revendication 4, caractérisé en ce que le cation métallique est le Pb ou le Ta.

7. Procédé selon l'une quelconque des revendications précédentes caractérisé en ce que le cation métallique est mis en œuvre sous forme d'un oxyde ou d'un sel.

8. Procédé selon l'une quelconque des revendications précédentes, caractérisé en ce que l'anion est mis en œuvre sous forme de l'acide hydrogéné correspondant ou de l'halogénure des métaux des colonnes IB, IIB, IVA, VA et VB et IA et IIA.

9. Procédé selon la revendication 8, caractérisé en ce que l'anion est mis en œuvre sous forme de l'acide hydrogéné correspondant.

10. Procédé selon l'une quelconque des revendications précédentes, caractérisé en ce que la pression totale est comprise entre 5 et 30 bars.

11. Procédé selon l'une quelconque des revendications précédentes, caractérisé en ce que le métal catalyseur est mis en œuvre sous forme métallique, d'un oxyde ou d'un sel ou d'un mélange d'entre eux.

12. Procédé selon la revendication 6, caractérisé en ce que le catalyseur est déposé sur support choisi parmi le charbon, la silice et l'alumine.

13. Procédé selon l'une quelconque des revendications précédentes, caractérisé en ce que l'on met en œuvre moins de 10 % en poids de catalyseur exprimés en métal par rapport au perfluoroacide aliphatique.

14. Procédé selon l'une quelconque des revendications précédentes, caractérisé en ce que le perfluoroacide aliphatique mis en œuvre est l'acide trifluoroacétique de formule $CF_3COOH$.

## Claims

1. Process for preparing 1,2-dihydroperfluoroalkanols by hydrogenation of the corresponding aliphatic perfluoroacids, characterized in that an acid of formula :

$$CnF_{2n+1}COOH$$

in which n is greater than or equal to 1, is reacted with hydrogen in the presence of a catalyst based on ruthenium, rhodium, iridium or platinum at at total pressure of between 1 and 50 bars, at a temperature of between 20° and 200 °C and in the presence of at least one additive chosen from the group consisting of halide anions and metal cations of groups IB, IIB, IVA, VA and VB of the Periodic Classification of the elements.

2. Process according to Claim 1, characterized in that the amount of additive used is between 0.001 and 10 gram-atoms per gram-atom of catalytic metal.

3. Process according to Claim 2, characterized in that the amount of additive is between 0.01 and 1 gram-atom.

4. Process according to any one of the preceding claims, characterized in that the metal cation is chosen from the cations of metals of groups IB, IIB, IVA, VA and VB of the Periodic Classification.

5. Process according to Claim 4, characterised in that the metal cation is chosen from Cu, Ag, Cd, Hg, Sn, Pb, Sb, Bi and Ta.

6. Process according to Claim 4, characterized in that the metal cation is Pb or Ta.

7. Process according to any one of the preceding claims, characterised in that the metal cation is employed in the form of an oxide or a salt.

8. Process according to any one of the preceding claims, characterized in that the anion is employed in the form of the corresponding hydracid or the halide of metals of groups IB, IIB, IVA, VA and VB, and IA and IIA.

9. Process according to Claim 8, characterized in that the anion is employed in the form of the corresponding hydracid.

10. Process according to any one of the preceding claims, characterized in that the total pressure is between 5 and 30 bars.

11. Process according to any one of the preceding claims, characterized in that the catalytic metal is employed in the form of the metal, an oxide or a salt, or a mixture of these.

12. Process according to Claim 6, characterized in that the catalyst is deposited on a support chosen from charcoal, silica and alumina.

13. Process according to any one of the preceding claims, characterized in that less than 10 % by weight of catalyst, expressed as metal relative to the aliphatic perfluoroacid, is employed.

14. Process according to any one of the preceding claims, characterized in that the aliphatic perfluoroacid employed is trifluoroacetic acid, of formula $CF_3COOH$.

## Patentansprüche

1. Verfahren zur Herstellung von 1,1-Dihydroperfluoralkanolen durch Hydrierung der entsprechenden aliphatischen Perfluorsäuren, dadurch gekennzeichnet, daß man eine Säure der Formel :

$$CnF_{2n+1}COOH$$

in der n größer oder gleich 1 ist, mit Wasserstoff in Gegenwart eines Katalysators auf Basis von Ruthenium, Rhodium, Iridium oder Platin unter einem Gesamtdruck zwischen 1 und 50 bar bei einer Temperatur zwischen 20° und 200 °C und in Gegenwart von wenigstens einem Additiv umsetzt, das aus der Gruppe ausgewählt ist, die die Halogenidanionen und die metallischen Kationen der Gruppen IB, IIB, IVA, VA und VB des Periodensystems enthält.

2. Verfahren nach Anspruch 1, dadurch gekennzeichnet, daß man eine Menge des Additivs zwischen 0,001 und 10 Grammatom pro Grammatom des Katalysatormetalls einsetzt.

3. Verfahren nach Anspruch 2, dadurch gekennzeichnet, daß die Menge des Additivs zwischen 0,01 und 1 Grammatom beträgt.

4. Verfahren nach einem der Ansprüche 1 bis 3, dadurch gekennzeichnet, daß das Metallkation ausgewählt wird unter den Kationen der Metalle der Gruppen IB, IIB, IVA, VA und VB des Periodensystems.

5. Verfahren nach Anspruch 4, dadurch gekennzeichnet, daß das Metallkation unter Cu, Ag, Cd, Hg, Sn, Pb, Sb, Bi und Ta ausgewählt wird.

6. Verfahren nach Anspruch 4, dadurch gekennzeichnet, daß das Metallkation das Pb oder Ta ist.

7. Verfahren nach einem der vorangehenden Ansprüche, dadurch gekennzeichnet, daß das Metallkation in Form eines Oxids oder eines Salzes eingesetzt wird.

8. Verfahren nach einem der vorangehenden Ansprüche, dadurch gekennzeichnet, daß das Anion in Form der entsprechenden Wasserstoffsäure oder der Halogenide der Metalle der Gruppen IB, IIB, IVA, VA, VB, IA und IIA eingesetzt wird.

9. Verfahren nach Anspruch 8, dadurch gekennzeichnet, daß das Anion in Form der entsprechenden Wasserstoffsäure eingesetzt wird.

10. Verfahren nach einem der vorangehenden Ansprüche, dadurch gekennzeichnet, daß der Gesamtdruck zwischen 5 und 30 bar beträgt.

11. Verfahren nach einem der vorangehenden Ansprüche, dadurch gekennzeichnet, daß das Katalysatormetall in metallischer Form, in Form eines Oxids oder eines Salzes oder eines Gemischs dieser Formen untereinander eingesetzt wird.

12. Verfahren nach Anspruch 6, dadurch gekennzeichnet, daß der Katalysator auf einem Träger abgelagert wird, der unter Kohle, Siliciumdioxid und Aluminiumoxid ausgewählt wird.

13. Verfahren nach einem der vorangehenden Ansprüche, dadurch gekennzeichnet, daß man weniger als 10 Gewichtsprozent Katalysator, ausgedrückt als Metall und bezogen auf die aliphatische Perfluorsäure, einsetzt.

14. Verfahren nach einem der vorangehenden Ansprüche, dadurch gekennzeichnet, daß die eingesetzte aliphatische Perfluorsäure Trifluoressigsäure der Formel $CF_3COOH$ ist.